# EUROPEAN PATENT SPECIFICATION

(11) **EP 3 487 881 B1**
(45) Date of publication and mention of the grant of the patent: **21.08.2024**
(21) Application number: 17754796.5
(22) Date of filing: 18.07.2017
(51) Int. Cl.: C07K 16/24, A61P 17/06

(54) **METHODS OF TREATING NEW-ONSET PLAQUE TYPE PSORIASIS USING IL-17 ANTAGONISTS**
VERFAHREN ZUR BEHANDLUNG EINER NEU AUSGEBROCHENEN PLAQUE-PSORIASIS MITHILFE VON IL-17-ANTAGONISTEN
PROCÉDÉS DE TRAITEMENT DU PSORIASIS DE TYPE EN PLAQUES D'APPARITION RÉCENTE AU MOYEN D'ANTAGONISTES DE L'IL-17

(30) Priority: 19.07.2016 US 201662364007 P
(43) Date of publication of application: 29.05.2019
(73) Proprietor: Novartis AG, 4056 Basel (CH)
(72) Inventor: FASTH, Andreas, 183 11 Taby Stockholm (SE); OLIVER, Jaime, 4002 Basel (CH)
(74) Representative: Carpmaels & Ransford LLP
(86) International application number: PCT/IB2017/054333
(87) International publication number: WO 2018/015880

(56) References cited:
- WO-A1-2016/103153
- RICHARD G. LANGLEY ET AL: "Secukinumab in Plaque Psoriasis - Results of Two Phase 3 Trials", NEW ENGLAND JOURNAL OF MEDICINE, THE - NEJM -, vol. 371, no. 4, 24 July 2014 (2014-07-24), US, pages 326 - 338, XP055414280, ISSN: 0028-4793, DOI: 10.1056/NEJMoa1314258
- DIAMANT THACI ET AL: "Secukinumab is superior to ustekinumab in clearing skin of subjects with moderate to severe plaque psoriasis: CLEAR, a randomized controlled trial", JOURNAL OF THE AMERICAN ACADEMY OF DERMATOLOGY., vol. 73, no. 3, 1 September 2015 (2015-09-01), US, pages 400 - 409, XP055387207, ISSN: 0190-9622, DOI: 10.1016/j.jaad.2015.05.013
- S. CHEUK ET AL: "Epidermal Th22 and Tc17 Cells Form a Localized Disease Memory in Clinically Healed Psoriasis", THE JOURNAL OF IMMUNOLOGY, vol. 192, no. 7, 1 April 2014 (2014-04-01), US, pages 3111 - 3120, XP055414273, ISSN: 0022-1767, DOI: 10.4049/jimmunol.1302313
- GRIFFITHS CHRISTOPHER E M ET AL: "Comparison of ixekizumab with etanercept or placebo in moderate-to-severe psoriasis (UNCOVER-2 and UNCOVER-3): results from two phase 3 randomised trials", LANCET (NORTH AMERICAN EDITION), vol. 386, no. 9993, August 2015 (2015-08-01), pages 541 - 551, XP002774596
- HARTWIG TOM ET AL: "Dermal IL-17-producing [gamma][delta] T cells establish long-lived memory in the skin.", EUROPEAN JOURNAL OF IMMUNOLOGY NOV 2015, vol. 45, no. 11, November 2015 (2015-11-01), pages 3022 - 3033, XP002774597, ISSN: 1521-4141
- WHARTON KEITH JR ET AL: "Secukinumab Reverses Disease-Defining Psoriasis Histopathology While Retaining Full T-cell Activation Potential", THE FASEB JOURNAL, FEDERATION OF AMERICAN SOCIETIES FOR EXPERIMENTAL BIOLOGY, US, vol. 29, no. Suppl. 1, 31 March 2015 (2015-03-31), pages LB443, XP009500504, ISSN: 0892-6638
- R. A. CLARK: "Resident memory T cells in human health and disease", SCIENCE TRANSLATIONAL MEDICINE, vol. 7, no. 269, 7 January 2015 (2015-01-07), pages 269rv1 - 269rv1, XP055412634, ISSN: 1946-6234, DOI: 10.1126/scitranslmed.3010641

## Description

### TECHNICAL FIELD

The present disclosure relates to secukinumab for use in methods for treating new-onset plaque-type psoriasis patients and inhibiting psoriasis disease progression in these patients.

### BACKGROUND OF THE DISCLOSURE

Psoriasis is an immune-mediated inflammatory disease that may have a major impact on a patient's life, especially when the intensity is moderate or severe. Treatment of psoriasis during the first years is typically conservative and frequently based on topical agents which rarely clear lesions completely. Treatment with systemic agents, including biologicals, is often initiated only when topical agents, phototherapy and conventional systemic treatment have proved to be inadequate, even in patients with moderate to severe disease. (Maza et al (2012) Br J Dermatol; 167(3):643-8.).

Psoriatic skin lesions are a "riot" of disorder, featuring dense inflammatory cell infiltrates, massive proliferation, impaired differentiation of the epidermis, formation of new blood vessels, and alterations in lymphatic structures. With effective therapy of chronic psoriasis, there is resolution of epidermal thickness, reduced numbers of inflammatory cells, and return of previously affected skin to a clinically normal state. Once therapy is discontinued, however, psoriatic skin lesions tend to recur, usually at the same sites that were previously affected, but sometimes also appearing at new sites.

Therefore, there is a need for a patient-centered therapeutic approach, undertaken early in the psoriasis treatment pathway (early intervention) with the goal of complete clearance, which may improve control of cutaneous symptoms and modify the course of the disease and the associated burden.

DIAMANT THACI ET AL, JOURNAL OF THE AMERICAN ACADEMY OF DERMATOLOGY., US, 2015, vol. 73, no. 3, pages 400-409, describes a study (NCT02074982) wherein subjects with moderate to severe plaque psoriasis received subcutaneous injection of secukinumab 300 mg or ustekinumab. GRIFFITHS CHRISTOPHER E M ET AL, LANCET (NORTH AMERICAN EDITION), 2015, vol. 386, no. 9993, pages 541-551 compares ixekizumab with etanercept or placebo in moderate-to-severe psoriasis. HARTWIG TOM ET AL, EUROPEAN JOURNAL OF IMMUNOLOGY NOV 2015, vol. 45, no. 11, pages 3022-3033, notes that dermal IL-17-producing [gamma] [delta] T cells establish long-lived memory in the skin. WHARTON KEITH JR ET AL, THE FASEB JOURNAL, FEDERATION OF AMERICAN SOCIETIES FOR EXPERIMENTAL BIOLOGY, US, 2015, vol. 29, no. Suppl. 1, describes that secukinumab reverses psoriasis histopathology. WO2016103153 is directed to pharmaceutical products and stable liquid compositions of IL-17 antibodies and antigen-binding fragments thereof, e.g., secukinumab.

### SUMMARY OF THE DISCLOSURE

Following effective therapy of chronic psoriasis, a subclinical inflammation may continue in psoriatic skin that may become clinical once treatment is stopped, e.g., during or after a "drug holiday". A possible explanation for this phenomenon can be found in the identification of a T-cell subset called tissue resident memory T cells (Trm) that has been proven to contribute to a tissue-localized immunological memory to viral infections of the skin (e.g., infections with herpes simplex virus). This subtype of T cell may be a key player for generating chronicity of immune-mediated inflammatory or auto-reactive disorders. (Clark, R. (2015) Sci Transl Med;7(269):269rv1).

A newly published study identifies Trm cells in the lesional skin of patients with psoriasis, which remain in the skin after resolution of lesions during treatment with biologic therapy (Cheuk et al (2014) J Immunol; 192(7):3111-20). The presence of these cells in the skin may explain the chronicity of the disease and the reoccurrence of relapses to the same anatomic locations. Cheuk et al (2014) also showed that Trm cells in the psoriatic plaques produce interleukin (IL)-17A and IL-22 upon activation, supporting the notion of Trm cells in psoriasis as Th17 cells with IL-17A as a key effector cytokine. In addition to the direct pathogenic effect of IL-17A from T cells on keratinocytes, IL-17A is also released by granulocytes and mast cells and plays an important role in early attracting more immune cells to the target organ. Therefore, inhibition of IL-17A early after disease onset is a novel and important therapeutic approach to interfering with the immune system before the establishment of extensive and chronic inflammation. This would be achieved by early blocking of the recruitment of inflammatory cells to the skin, including Th17, as well as by blocking the key effector functions of Trm cells.

Secukinumab is the first IL-17A inhibitor approved for the treatment of psoriasis in patients requiring systemic treatment. Early treatment of psoriasis patients with IL-17 antagonists, such as secukinumab, in new-onset moderate to severe psoriasis is expected to block recruitment of inflammatory cells and antagonize the effect of IL-17A produced by a subset of T cells. The expected clinical outcome is a change in the natural course of the disease to a milder state by hindering spreading of psoriasis (Trm cells) to new anatomical locations or ultimately totally hindering reoccurrence of new lesions, i.e., inducing minimal, or no, disease activity.

Accordingly, disclosed herein is secukinumab for use in methods of treating moderate to severe new-onset plaque-type psoriasis, characterized in that 150 mg - 300 mg secukinumab is to be subcutaneously administered to a patient in need thereof at weeks 0, 1, 2, 3, and 4, followed by monthly dosing; wherein the first psoriasis plaque in the patient appeared within 12 months prior to the start of the treatment. The invention is set out in the appended claims.

### BRIEF DESCRIPTON OF THE FIGURES

**Figure 1****.** Treatment with anti-IL-17 Ab, dosed at 100mg/kg i.p. on days -3, 0, 4, 7 and 11, inhibited skin swelling AUC_{[0-14 days]} by 22.94±12.25%.
**Figure 2****.** Treatment with anti-IL-17 Ab, dosed at 100mg/kg s.c. on day -3 only, inhibited the ear swelling AUC_{[0-10 days]} by 25.50±2.28%. * p<0.05, unpaired t-test.
**Figure 3****.** Treatment with anti-IL-17 Ab, dosed at 30mg/kg i.p. on day -1 only, inhibited the ear swelling AUC_{[0-8 days]} by 38.57±3.98%. *** p<0.001, paired t-test.

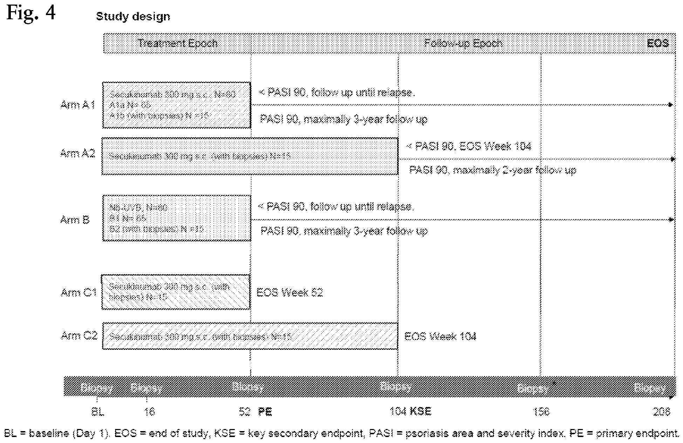

**Figure 4****.** CAIN457A2322 Trial Design
**Figure 5****.** Shows percentages of PASI 75, PASI 90, PASI 100 and IGA mod 2011 0 or 1 response (non-responder imputation) at week 52 by disease duration (Full analysis set). Clinical Trials: CAIN457A2302 and CAIN457A2303; Treatment: secukinumab 150mg.
**Figure 6****.** Shows percentages of PASI 75, PASI 90, PASI 100 and IGA mod 2011 0 or 1 response (non-responder imputation) at week 52 by disease duration (Full analysis set). Clinical Trials: CAIN457A2302 and CAIN457A2303; Treatment: secukinumab 300 mg.

### DETAILED DESCRIPTION OF THE DISCLOSURE

As used herein, IL-17 refers to interleukin-17A (IL-17A).

The term "comprising" encompasses "including" as well as "consisting," e.g., a composition "comprising" X may consist exclusively of X or may include something additional, e.g., X + Y.

The term "about" in relation to a numerical value x means, for example, +/-10%. When used in front of a numerical range or list of numbers, the term "about" applies to each number in the series, e.g., the phrase "about 1-5" should be interpreted as "about 1 - about 5", or, e.g., the phrase "about 1, 2, 3, 4" should be interpreted as "about 1, about 2, about 3, about 4, etc."

The word "substantially" does not exclude "completely," e.g., a composition which is "substantially free" from Y may be completely free from Y. Where necessary, the word "substantially" may be omitted from the definition of the disclosure.

The term "antibody" as referred to herein includes naturally-occurring and whole antibodies. A naturally-occurring "antibody" is a glycoprotein comprising at least two heavy (H) chains and two light (L) chains inter-connected by disulfide bonds. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, CL. The V_{H} and V_{L} regions can be further subdivided into regions of hypervariability, termed hypervariable regions or complementarity determining regions (CDR), interspersed with regions that are more conserved, termed framework regions (FR). Each V_{H} and V_{L} is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. Exemplary antibodies include secukinumab (**Table 1**), antibody XAB4 (US Patent No. 9,193,788), and ixekizumab (U.S. Patent No. 7,838,638).

The term "antigen-binding fragment" of an antibody, as used herein, refers to fragments of an antibody that retain the ability to specifically bind to an antigen (e.g., IL-17). It has been shown that the antigen-binding function of an antibody can be performed by fragments of a full-length antibody. Examples of binding fragments encompassed within the term "antigen-binding portion" of an antibody include a Fab fragment, a monovalent fragment consisting of the V_{L}, V_{H}, CL and CH1 domains; a F(ab)2 fragment, a bivalent fragment comprising two Fab fragments linked by a disulfide bridge at the hinge region; a Fd fragment consisting of the V_{H} and CH1 domains; a Fv fragment consisting of the V_{L} and V_{H} domains of a single arm of an antibody; a dAb fragment (Ward et al., 1989 Nature 341:544-546), which consists of a V_{H} domain; and an isolated CDR. Exemplary antigen-binding fragments include the CDRs of secukinumab as set forth in SEQ ID NOs: 1-6 and 11-13 (**Table 1**), preferably the heavy chain CDR3. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see, e.g., Bird et al., 1988 Science 242:423-426; and Huston et al., 1988 Proc. Natl. Acad. Sci. 85:5879-5883). Such single chain antibodies are also intended to be encompassed within the term "antibody". Single chain antibodies and antigen-binding portions are obtained using conventional techniques known to those of skill in the art.

An "isolated antibody", as used herein, refers to an antibody that is substantially free of other antibodies having different antigenic specificities (e.g., an isolated antibody that specifically binds IL-17 is substantially free of antibodies that specifically bind antigens other than IL-17). The term "monoclonal antibody" or "monoclonal antibody composition" as used herein refer to a preparation of antibody molecules of single molecular composition. The term "human antibody", as used herein, is intended to include antibodies having variable regions in which both the framework and CDR regions are derived from sequences of human origin. A "human antibody" need not be produced by a human, human tissue or human cell. The human antibodies of the disclosure may include amino acid residues not encoded by human sequences (e.g., mutations introduced by random or site-specific mutagenesis *in vitro,* by N-nucleotide addition at junctions *in vivo* during recombination of antibody genes, or by somatic mutation *in vivo*).

The term "IL-17" refers to IL-17A, formerly known as CTLA8, and includes wild-type IL-17A from various species (e.g., human, mouse, and monkey), polymorphic variants of IL-17A, and functional equivalents of IL-17A. Functional equivalents of IL-17A according to the present disclosure preferably have at least about 65%, 75%, 85%, 95%, 96%, 97%, 98%, or even 99% overall sequence identity with a wild-type IL-17A (e.g., human IL-17A), and substantially retain the ability to induce IL-6 production by human dermal fibroblasts.

The term "K_{D}" is intended to refer to the dissociation rate of a particular antibody-antigen interaction. The term "K_{D}", as used herein, is intended to refer to the dissociation constant, which is obtained from the ratio of K_{d} to Kₐ (i.e., K_{d}/Kₐ) and is expressed as a molar concentration (M). K_{D} values for antibodies can be determined using methods well established in the art. A preferred method for determining the K_{D} of an antibody is by using surface plasmon resonance, or using a biosensor system such as a Biacore^{®} system. The IL-17 antibody or antigen-binding fragment thereof, e.g., secukinumab, may bind human IL-17 with a K_{D} of about 100-250 pM.

The term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity. Standard assays to evaluate the binding affinity of the antibodies toward IL-17 of various species are known in the art, including for example, ELISAs, western blots and RIAs. The binding kinetics (e.g., binding affinity) of the antibodies also can be assessed by standard assays known in the art, such as by Biacore analysis.

An antibody that "inhibits" one or more of these IL-17 functional properties (e.g., biochemical, immunochemical, cellular, physiological or other biological activities, or the like) as determined according to methodologies known to the art and described herein, will be understood to relate to a statistically significant decrease in the particular activity relative to that seen in the absence of the antibody (or when a control antibody of irrelevant specificity is present). An antibody that inhibits IL-17 activity affects a statistically significant decrease, e.g., by at least about 10% of the measured parameter, by at least 50%, 80% or 90%, or greater than 95%, 98% or 99% of IL-17 functional activity.

"Inhibit IL-6" as used herein refers to the ability of an IL-17 antibody or antigen-binding fragment thereof (e.g., secukinumab) to decrease IL-6 production from primary human dermal fibroblasts. The production of IL-6 in primary human (dermal) fibroblasts is dependent on IL-17 (Hwang et al., (2004) Arthritis Res Ther; 6:R120-128). In short, human dermal fibroblasts are stimulated with recombinant IL-17 in the presence of various concentrations of an IL-17 binding molecule or human IL-17 receptor with Fc part. The chimeric anti-CD25 antibody Simulect^{®} (basiliximab) may be conveniently used as a negative control. Supernatant is taken after 16 h stimulation and assayed for IL-6 by ELISA. An IL-17 antibody or antigen-binding fragment thereof, e.g., secukinumab, typically has an IC₅₀ for inhibition of IL-6 production (in the presence 1 nM human IL-17) of about 50 nM or less (e.g., from about 0.01 to about 50 nM) when tested as above, i.e., said inhibitory activity being measured on IL-6 production induced by hu-IL-17 in human dermal fibroblasts. IL-17 antibodies or antigen-binding fragments thereof, e.g., secukinumab, and functional derivatives thereof may have an IC₅₀ for inhibition of IL-6 production as defined above of about 20 nM or less, more preferably of about 10 nM or less, more preferably of about 5 nM or less, more preferably of about 2 nM or less, more preferably of about 1 nM or less.

The term "derivative", unless otherwise indicated, is used to define amino acid sequence variants, and covalent modifications (e.g., pegylation, deamidation, hydroxylation, phosphorylation, methylation, etc.) of an IL-17 antibody or antigen-binding fragment thereof, e.g., secukinumab, according to the present disclosure, e.g., of a specified sequence (e.g., a variable domain). A "functional derivative" includes a molecule having a qualitative biological activity in common with the disclosed IL-17 antibodies. A functional derivative includes fragments and peptide analogs of an IL-17 antibody as disclosed herein. Fragments comprise regions within the sequence of a polypeptide according to the present disclosure, e.g., of a specified sequence. Functional derivatives of the IL-17 antibodies disclosed herein (e.g., functional derivatives of secukinumab) preferably comprise V_{H} and/or V_{L} domains that have at least about 65%, 75%, 85%, 95%, 96%, 97%, 98%, or even 99% overall sequence identity with the V_{H} and/or V_{L} sequences of the IL-17 antibodies and antigen-binding fragments thereof disclosed herein (e.g., the V_{H} and/or V_{L} sequences of **Table 1)**, and substantially retain the ability to bind human IL-17 or, e.g., inhibit IL-6 production of IL-17 induced human dermal fibroblasts.

The phrase "substantially identical" means that the relevant amino acid or nucleotide sequence (e.g., V_{H} or V_{L} domain) will be identical to or have insubstantial differences (e.g., through conserved amino acid substitutions) in comparison to a particular reference sequence. Insubstantial differences include minor amino acid changes, such as 1 or 2 substitutions in a 5 amino acid sequence of a specified region (e.g., V_{H} or V_{L} domain). In the case of antibodies, the second antibody has the same specificity and has at least 50% of the affinity of the same. Sequences substantially identical (e.g., at least about 85% sequence identity) to the sequences disclosed herein are also part of this application. The sequence identity of a derivative IL-17 antibody (e.g., a derivative of secukinumab, e.g., a secukinumab biosimilar antibody) can be about 90% or greater, e.g., 90%, 91%, 92%, 93%, 94%, 95%, 96%, 97%, 98%, 99% or higher relative to the disclosed sequences.

"Identity" with respect to a native polypeptide and its functional derivative is defined herein as the percentage of amino acid residues in the candidate sequence that are identical with the residues of a corresponding native polypeptide, after aligning the sequences and introducing gaps, if necessary, to achieve the maximum percent identity, and not considering any conservative substitutions as part of the sequence identity. Neither N- or C-terminal extensions nor insertions shall be construed as reducing identity. Methods and computer programs for the alignment are well known. The percent identity can be determined by standard alignment algorithms, for example, the Basic Local Alignment Search Tool (BLAST) described by Altshul et al. ((1990) J. Mol. Biol., 215: 403 410); the algorithm of Needleman et al. ((1970) J. Mol. Biol., 48: 444 453); or the algorithm of Meyers et al. ((1988) Comput. Appl. Biosci., 4: 11 17). A set of parameters may be the Blosum 62 scoring matrix with a gap penalty of 12, a gap extend penalty of 4, and a frameshift gap penalty of 5. The percent identity between two amino acid or nucleotide sequences can also be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.
"Amino acid(s)" refer to all naturally occurring L-α-amino acids, e.g., and include D-amino acids. The phrase "amino acid sequence variant" refers to molecules with some differences in their amino acid sequences as compared to the sequences according to the present disclosure. Amino acid sequence variants of an antibody according to the present disclosure, e.g., of a specified sequence, still have the ability to bind the human IL-17 or, e.g., inhibit IL-6 production of IL-17 induced human dermal fibroblasts. Amino acid sequence variants include substitutional variants (those that have at least one amino acid residue removed and a different amino acid inserted in its place at the same position in a polypeptide according to the present disclosure), insertional variants (those with one or more amino acids inserted immediately adjacent to an amino acid at a particular position in a polypeptide according to the present disclosure) and deletional variants (those with one or more amino acids removed in a polypeptide according to the present disclosure).

The term "pharmaceutically acceptable" means a nontoxic material that does not interfere with the effectiveness of the biological activity of the active ingredient(s).

The term "administering" in relation to a compound, e.g., an IL-17 binding molecule or another agent, is used to refer to delivery of that compound to a patient by any route.

As used herein, a "therapeutically effective amount" refers to an amount of an IL-17 antagonist, e.g., IL-17 binding molecule (e.g., IL-17 antibody or antigen-binding fragment thereof, e.g., secukinumab) or IL-17 receptor binding molecule (e.g., IL-17 antibody or antigen-binding fragment thereof) that is effective, upon single or multiple dose administration to a patient (such as a human) for treating, preventing, preventing the onset of, curing, delaying, reducing the severity of, ameliorating at least one symptom of a disorder or recurring disorder, or prolonging the survival of the patient beyond that expected in the absence of such treatment. When applied to an individual active ingredient (e.g., an IL-17 antagonist, e.g., secukinumab) administered alone, the term refers to that ingredient alone. When applied to a combination, the term refers to combined amounts of the active ingredients that result in the therapeutic effect, whether administered in combination, serially or simultaneously.

The term "treatment" or "treat" is herein defined as the application or administration of an IL-17 antibody according to the disclosure, for example, secukinumab or ixekizumab, or a pharmaceutical composition comprising said anti-IL-17 antibody, to a subject or to an isolated tissue or cell line from a subject, where the subject has a particular disease (e.g., psoriasis), a symptom associated with the disease (e.g., psoriasis), or a predisposition towards development of the disease (e.g., psoriasis), where the purpose is to cure (if applicable), delay the onset of, reduce the severity of, alleviate, ameliorate one or more symptoms of the disease, improve the disease, reduce or improve any associated symptoms of the disease or the predisposition toward the development of the disease. The term "treatment" or "treat" includes treating a patient suspected to have the disease as well as patients who are ill or who have been diagnosed as suffering from the disease or medical condition, and includes suppression of clinical relapse.

As used herein, the phrase "new-onset plaque-type psoriasis" is used to mean plaque-type psoriasis in which the first psoriasis plaque appeared (debuted) within 12 months (≤ 12 months) prior to the start of treatment with the IL-17 antagonist (e.g., secukinumab).

As used herein, the phrases "has not been previously treated with a systemic treatment for psoriasis" and "naive" refer to a psoriasis patient who has not been previously treated with a systemic agent, e.g., methotrexate, cyclosporine, a biological (e.g., ustekinumab, infliximab, TNF alpha inhibitors, etc.), etc., for psoriasis. Systemic agents (i.e., agents given orally, by injection, etc.) differ from local agents (e.g., topicals and phototherapy) in that systemic agents have a systemic effect when delivered to a patient. As used herein, the phrase "has not been previously treated with phototherapy for psoriasis" refers to a psoriasis patient who has not been previously treated with narrow band-UVB (nb-UVB) for psoriasis. In some embodiments, the patient has not been previously treated with a systemic treatment for psoriasis. In some embodiments, the patient has not been previously treated with phototherapy for psoriasis.

As used herein, the phrases "has been previously treated with a systemic agent for psoriasis" is used to mean a patient that has previously undergone psoriasis treatment using a systemic agent. Such patients include those previously treated with biologics, such as ustekinumab, and those previously treated with non-biologics, such as cyclosporine. In some embodiments of the disclosure, the patient has been previously treated with a systemic agent for psoriasis.

In some embodiments, the patient has been previously treated with a systemic agent for psoriasis (e.g., methotrexate, cyclosporine), but the patient has **not** been previously treated with a systemic biological drug (i.e., a drug produced by a living organisim, e.g., antibodies, receptor decoys, etc.) for psoriasis (e.g., ustekinumab, ixekizumab, broadalumab, TNF alpha inhibitors (etanercept, adalimumab, remicade, etc.), secukinumab, etc.). In this case, the patient is referred to as "biological-naive." In preferred embodiments, the patient is biological-naive.

As used herein, the term "TNF failure" refers to a patient who had an inadequate response to or was intolerant to prior treatment with a TNF alpha antagonist (e.g., etanercept, adalimumab, etc.). A patient who has responded adequately to prior treatment with a TNF alpha antagonist (e.g., etanercept, adalimumab, etc.) but has discontinued due to a side effect is termed "intolerant". TNF failures are also sometimes referred to as "TNF-IR" patients. In some embodiments, prior to administering the IL-17 antagonist, the patient is a TNF failure.

As used herein, "selecting" and "selected" in reference to a patient is used to mean that a particular patient is specifically chosen from a larger group of patients on the basis of (due to) the particular patient having a predetermined criteria. Similarly, "selectively treating" refers to providing treatment to a patient having a particular disease, where that patient is specifically chosen from a larger group of patients on the basis of the particular patient having a predetermined criterion. Similarly, "selectively administering" refers to administering a drug to a patient that is specifically chosen from a larger group of patients on the basis of (due to) the particular patient having a predetermined criterion. By selecting, selectively treating and selectively administering, it is meant that a patient is delivered a personalized therapy based on the patient's personal history (e.g., prior therapeutic interventions, e.g., prior treatment with biologics), biology (e.g., particular genetic markers), and/or manifestation (e.g., not fulfilling particular diagnostic criteria), rather than being delivered a standard treatment regimen based solely on the patient's membership in a larger group. Selecting, in reference to secukinumab for use in a method of treatment as used herein, does not refer to fortuitous treatment of a patient having a particular criterion, but rather refers to the deliberate choice to administer treatment to a patient based on the patient having a particular criterion. Thus, selective treatment/administration differs from standard treatment/administration, which delivers a particular drug to all patients having a particular disease, regardless of their personal history, manifestations of disease, and/or biology. The patient is selected for treatment based on having new-onset plaque-type psoriasis.

As used herein, the phrase "tissue resident memory T cells in the skin" refers to non-recirculating memory T cells that persist in the epidermis. (See, e.g., Clark (2015), *supra).* In some embodiments of the disclosure, treatment with the IL-17 antagonist (secukinumab) reduces tissue resident memory T-cells in the skin.

As used herein, the phrase "subset effector T cells producing interleukin -17 (IL-17) and/or interleukin -22 (IL-22) in the skin" means epidermal CD4 and CD8 T cells that produce IL-17 and IL-22. (See, e.g., Clark (2015), *supra).* In some embodiments of the disclosure, treatment with the IL-17 antagonist (secukinumab) reduces subset effector T-cells producing interleukin - 17 (IL-17) and/or interleukin -22 (IL-22) in the skin.

As used herein, the phrase "regulatory T cells in the skin" refers to a population of T cells, characterized by expression of the transcription factor Foxp3, found in skin. (See, e.g., Sanchez Rodriguez et al. (2014) J. Clin. Invest. 124(3): 1027-1036). In some embodiments of the disclosure, treatment with the IL-17 antagonist (secukinumab) reduces and normalizes the number of regulatory T cells in the skin.

As used herein, the phrase "dermal dendritic cell-T cell aggregates in the skin" refers to dermal clusters comprising dendritic cells and infiltrating T cells. (See, e.g., Kim et al. (2014) J. Invest. Derm. 134(5): 1462-65). In some embodiments of the disclosure, treatment with the IL-17 antagonist (secukinumab) reduces dermal dendritic cell-T cell aggregates in the skin.

Collectively, the acts of reducing tissue resident memory T-cells in the skin, reducing subset effector T-cells producing interleukin -17 (IL-17) and/or interleukin -22 (IL-22) in the skin, reducing regulatory T cells in the skin, and reducing dermal dendritic cell-T cell aggregates in the skin (e.g., reducing cell-mediated immunity in the skin, reducing T-cell-mediated immune response in the skin) is referred to herein as "modulating the immune mechanisms causing psoriasis disease chronicity." In some embodiments of the disclosure, treatment with the IL-17 antagonist (secukinumab) modulates the immune mechanisms causing psoriasis disease chronicity.

The plaque psoriasis disease course begins with small skin plaques ~ 1/8 of an inch wide, typically in the same areas on opposite sides of the body. They grow slowly and develop into thick, dry plaques. If the plaque is scratched or scraped, bleeding spots the sizes of pinheads appear underneath. This is known as the Auspitz sign. Some patches become annular, with a clear center and scaly raised borders. Eventually, separate patches join together to form larger areas. In some cases, the patches cover wide areas of the back or chest, termed geographic plaques. As used herein, the phrase "slowing psoriasis disease progression" means decelerating the advancement rate of the disease course of plaque-type psoriasis. In some embodiments of the disclosure, treatment with the IL-17 antagonist (secukinumab) slows psoriasis disease progression.

As used herein, the phrase "psoriasis flare" comprises the manifestation of plaque-type psoriasis, including plaques, irritated patches of skin, redness (e.g., particularly on elbows, knees, trunk and scalp), changes and/or disfiguration in nails, dandruff, and any combination thereof. Typically, a psoriasis flare includes the formation of psoriasis plaques. In some embodiments of the disclosure, treatment with the IL-17 antagonist (secukinumab) prevents psoriasis flares, decreases the severity of psoriasis flares, and/or decreases the frequency of psoriasis flares.

As used herein, the phrase "decreasing the severity of psoriasis flares" and the like means reducing the intensity of a psoriasis flare, e.g., reducing the percentage of skin affected by psoriasis, reducing the intensity of a particular flare component (e.g., reducing the number, size, thickness, etc. of plaques, reducing the extent of skin irritation, reducing scaling, reducing erythema, reducing changes and/or disfigurations in nails, reducing dandruff, etc.), and/or reducing the amount of time a flare (or component thereof) persists. The severity of a flare may be measured using various tools, e.g., the body surface area (BSA) test, the investigator's global assessment (IGA, IGA mod 2011), physicians global assessment (PGA), the psoriasis area and severity index (PASI), and patient reported outcomes, e.g., the Dermatology life quality index (DLQI) and the Work productivity and activity impairment questionnaire: psoriasis (WPAI:PSO). In some embodiments, the patient achieves at least a 50% reduction of the Psoriasis Area and Severity Index Score (PASI 50) at week 12 of treatment. In some embodiments, and uses, the patient achieves at least a 75% reduction of the Psoriasis Area and Severity Index Score (PASI 75) at week 12 of treatment. In some embodiments, and uses, the patient achieves at least a 90% reduction of the Psoriasis Area and Severity Index Score (PASI 90) at week 12 of treatment. In some embodiments, and uses, the patient achieves 100% reduction of the Psoriasis Area and Severity Index Score (PASI 100) at week 12 of treatment.

As used herein, the phrase "decreasing the frequency of psoriasis flares" and the like means reducing the incidence of psoriasis flares, e.g., reducing the incidence of plaques and/or other psoriasis flare components (e.g., plaques, skin irritation, scaling, erythema, changes in nails, dandruff, etc.). By decreasing the frequency of psoriasis flares, a patient will experience fewer psoriasis relapses. The incidence of flares may be assessed by monitoring a patient over time to determine if the prevalence of flares decreases.

As used herein, the phrase "preventing psoriasis flares" means eliminating future psoriasis flares and/or flare components.

Numerous psoriasis patients eventually progress to psoriatic arthritis (PsA). Treatment of new-onset psoriasis patients with an IL-17 antagonist (e.g., an IL-17 antibody, like secukinumab) is expected to decrease the likelihood that these patients will eventually develop PsA, cardiovascular disease, metabolic syndrome (diabetes mellitus, obesity), and other conditions. As used herein, the phrase "decreasing the likelihood that a psoriasis patient will develop psoriatic arthritis" refers to a reduction in the probability that a psoriasis patient will develop psoriatic arthritis. As used herein, the phrase "delaying the onset of psoriatic arthritis in a psoriasis patient" refers to postponing development of the signs and symptoms, and/or structural damage associated with PsA in a psoriasis patient. As used herein, the phrase "preventing progression from psoriasis to psoriatic arthritis in a psoriasis patient" refers to inhibiting the development of PsA in a psoriasis patient.

As used herein "mild psoriasis" is defined as psoriasis disease in which body surface area (BSA) ≤10 and psoriasis area and severity index (PASI) <10 and dermatology life quality index (DLQI) ≤10. As used herein, "moderate to severe psoriasis" is defined as psoriasis disease in which (BSA > 10 or PASI > 10) and DLQI > 10. *See* Mrowietz et al. (2011) Arch Dermatol Res. 303(1):1-10. In the invention, the patient has moderate to severe psoriasis. In some embodiments, long-term treatment with the IL-17 antagonist (secukinumab) converts a patient from having moderate to severe psoriasis to having mild psoriasis due to a modification of the psoriasis disease course.

### IL-17 Antagonists

The invention utilizes the IL-17 antagonist secukinumab.

Secukinumab comprises: an immunoglobulin V_{H} domain comprising the amino acid sequence set forth as SEQ ID NO: 8 and an immunoglobulin V_{L} domain comprising the amino acid sequence set forth as SEQ ID NO: 10. For ease of reference the amino acid sequences of the hypervariable regions of the secukinumab monoclonal antibody, based on the Kabat definition and as determined by the X-ray analysis and using the approach of Chothia and coworkers, is provided in **Table 1**, below.

**Table 1: Amino acid sequences of the hypervariable regions of secukinumab.**

| **Light-Chain** | | |
|---|---|---|
| CDR1' | Kabat | R-A-S-Q-S-V-S-S-S-Y-L-A (SEQ ID NO:4) |
| | Chothia | R-A-S-Q-S-V-S-S-S-Y-L-A (SEQ ID NO:4) |
| CDR2' | Kabat | G-A-S-S-R-A-T (SEQ ID NO:5) |
| | Chothia | G-A-S-S-R-A-T (SEQ ID NO:5) |
| CDR2' | Kabat | Q-Q-Y-G-S-S-P-C-T (SEQ ID NO:6) |
| | Chothia | Q-Q-Y-G-S-S-P-C-T (SEQ ID NO:6) |

| **Heavy-Chain** | | |
|---|---|---|
| CDR1 | Kabat | N-Y-W-M-N (SEQ ID NO:1) |
| CDR1-x | Chothia | G-F-T-F-S-N-Y-W-M-N (SEQ ID NO:11) |
| CDR2 | Kabat | A-I-N-Q-D-G-S-E-K-Y-Y-V-G-S-V-K-G (SEQ ID NO:2) |
| CDR2-x | Chothia | A-I-N-Q-D-G-S-E-K-Y-Y (SEQ ID NO:12) |
| CDR3 | Kabat | D-Y-Y-D-I-L-T-D-Y-Y-I-H-Y-W-Y-F-D-L (SEQ ID NO:3) |
| CDR3-x | Chothia | C-V-R-D-Y-Y-D-I-L-T-D-Y-Y-I-H-Y-W-Y-F-D-L-W-G (SEQ ID NO:13) |

The DNA encoding the VL of secukinumab is set forth in SEQ ID NO:9. The DNA encoding the V_{H} of secukinumab is set forth in SEQ ID NO:7.

Secukinumab comprises the three CDRs of SEQ ID NO:10 and the three CDRs of SEQ ID NO:8. CDRs of SEQ ID NO:8 and SEQ ID NO:10 may be found in **Table 1.** The free cysteine in the light chain (CysL97) may be seen in SEQ ID NO:6.
Secukinumab comprises the light chain of SEQ ID NO:14 and the heavy domain of SEQ ID NO:15. Secukinumab comprises the three CDRs of SEQ ID NO:14 and the three CDRs of SEQ ID NO:15. CDRs of SEQ ID NO:14 and SEQ ID NO:15 may be found in **Table 1.** The heavy chain framework of the secukinumab antibody consists in sequence of FR1 (amino acid 1 to 30 of SEQ ID NO:8), FR2 (amino acid 36 to 49 of SEQ ID NO:8), FR3 (amino acid 67 to 98 of SEQ ID NO:8) and FR4 (amino acid 117 to 127 of SEQ ID NO:8) regions. Taking into consideration the determined hypervariable regions of secukinumab by X-ray analysis, the heavy chain framework consists in sequence of FR1-x (amino acid 1 to 25 of SEQ ID NO:8), FR2-x (amino acid 36 to 49 of SEQ ID NO:8), FR3-x (amino acid 61 to 95 of SEQ ID NO:8) and FR4 (amino acid 119 to 127 of SEQ ID NO:8) regions. In a similar manner, the light chain framework consists, in sequence, of FR1' (amino acid 1 to 23 of SEQ ID NO:10), FR2' (amino acid 36 to 50 of SEQ ID NO:10), FR3' (amino acid 58 to 89 of SEQ ID NO:10) and FR4' (amino acid 99 to 109 of SEQ ID NO:10) regions.

Secukinumab is a recombinant high-affinity, fully human monoclonal anti-human interleukin-17A (IL-17A, IL-17) antibody of the IgG1/kappa isotype. Secukinumab (see, e.g., WO2006/013107 and WO2007/117749) has a very high affinity for IL-17, i.e., a K_{D} of about 100-200 pM and an IC₅₀ for *in vitro* neutralization of the biological activity of about 0.67 nM human IL-17A of about 0.4 nM. Thus, secukinumab inhibits antigen at a molar ratio of about 1:1. This high binding affinity makes the secukinumab antibody particularly suitable for therapeutic applications. Furthermore, it has been determined that secukinumab has a very long half-life, i.e., about 4 weeks, which allows for prolonged periods between administration, an exceptional property when treating chronic life-long disorders, such as psoriasis.

### Secukinumab for Use in Methods of Treatment for New-Onset Plaque-Type Psoriasis

Secukinumab may be incorporated into pharmaceutical compositions and administered *in vivo* to treat new-onset plaque-type psoriasis patients (e.g., human patients).

The patient is administered 150 mg -300 mg (e.g., preferably 150 mg or 300 mg) of secukinumab by subcutaneous (SC) injection at weeks 0, 1, 2, 3, and 4, followed by once monthly dosing.

Secukinumab may be used as a pharmaceutical composition when combined with a pharmaceutically acceptable carrier. Such a composition may contain, in addition to an IL-17 antagonist, carriers, various diluents, fillers, salts, buffers, stabilizers, solubilizers, and other materials well known in the art. The characteristics of the carrier will depend on the route of administration. The pharmaceutical compositions for use in the disclosed methods may also contain additional therapeutic agents for treatment of the particular targeted disorder. For example, a pharmaceutical composition may also include anti-inflammatory agents. Such additional factors and/or agents may be included in the pharmaceutical composition to produce a synergistic effect with the IL-17 binding molecules, or to minimize side effects caused by the IL-17 antagonists (secukinumab). In preferred embodiments, the pharmaceutical compositions for use in the disclosed methods comprise secukinumab at 150 mg/ml.

Pharmaceutical compositions for use in the disclosed methods may be manufactured in conventional manner. In one embodiment, the pharmaceutical composition is provided in lyophilized form. For immediate administration it is dissolved in a suitable aqueous carrier, for example sterile water for injection or sterile buffered physiological saline. If it is considered desirable to make up a solution of larger volume for administration by infusion rather than a bolus injection, may be advantageous to incorporate human serum albumin or the patient's own heparinised blood into the saline at the time of formulation. The presence of an excess of such physiologically inert protein prevents loss of antibody by adsorption onto the walls of the container and tubing used with the infusion solution. If albumin is used, a suitable concentration is from 0.5 to 4.5% by weight of the saline solution. Other formulations comprise liquid or lyophilized formulation.

Antibodies, e.g., antibodies to IL-17, are typically formulated either in aqueous form ready for parenteral administration or as lyophilisates for reconstitution with a suitable diluent prior to administration. In some embodiments of the disclosed methods and uses, secukinumab is formulated as a lyophilisate. Suitable lyophilisate formulations can be reconstituted in a small liquid volume (e.g., 2ml or less) to allow subcutaneous administration and can provide solutions with low levels of antibody aggregation. The use of antibodies as the active ingredient of pharmaceuticals is now widespread, including the products HERCEPTIN^{™} (trastuzumab), RITUXAN^{™} (rituximab), SYNAGIS^{™} (palivizumab), etc. Techniques for purification of antibodies to a pharmaceutical grade are well known in the art. When a therapeutically effective amount of the IL-17 antagonist secukinumab is administered by subcutaneous injection, the IL-17 antagonist will be in the form of a pyrogen-free, parenterally acceptable solution. A pharmaceutical composition for subcutaneous injection may contain, in addition to the IL-17 antagonist, an isotonic vehicle such as sodium chloride, Ringer's solution, dextrose, dextrose and sodium chloride, lactated Ringer's solution, or other vehicle as known in the art.

The appropriate dosage will vary depending upon, for example, the host, the mode of administration and the nature and severity of the condition being treated, and on the nature of prior treatments that the patient has undergone. Ultimately, the attending health care provider will decide the amount of the IL-17 antagonist with which to treat each individual patient. In some embodiments, the attending health care provider may administer low doses of the IL-17 antagonist and observe the patient's response. In other embodiments, the initial dose(s) of IL-17 antagonist administered to a patient are high, and then are titrated downward until signs of relapse occur. Larger doses of the IL-17 antagonist may be administered until the optimal therapeutic effect is obtained for the patient, and the dosage is not generally increased further.

In the invention, the IL-17 antagonist secukinumab is administered to a patient, e.g., a mammal (e.g., a human). While it is understood that the disclosed methods provide for treatment of new-onset plaque-type psoriasis patients using the IL-17 antagonist secukinumab, this does not preclude that, if the patient is to be ultimately treated with the IL-17 antagonist, such IL-17 antagonist therapy is necessarily a monotherapy. Indeed, if a patient is selected for treatment with the IL-17 antagonist, then the IL-17 antagonist (secukinumab) may be administered in accordance with the methods of the disclosure either alone or in combination with other agents and therapies for treating new-onset plaque-type psoriasis patients, e.g., in combination with at least one additional psoriasis agent. When coadministered with one or more additional psoriasis agent(s), the IL-17 antagonist may be administered either simultaneously with the other agent, or sequentially. If administered sequentially, the attending physician will decide on the appropriate sequence of administering the IL-17 antagonist in combination with other agents and the appropriate dosages for co-delivery.

Various therapies may be beneficially combined with secukinumab, during treatment of new-onset plaque psoriasis. Such therapies include topicals (over the counter, non-steroidal compounds, and steroidal compound), phototherapy and systemic treatment (e.g., with biologicals or chemical entities).

Non-limiting examples of topical psoriasis agents for use with secukinumab include salicylic acid, coal tar, Dovonex^{®} (calcipotriene), Taclonex^{®} (calcipotriene and betamethasone dipropionate), Tazorec^{®} (tazarotene), pimecrolimus, tacrolimus, Vectical^{®} (calcitriol), Zithranol-RR^{®} (anthralin) and topical steroids (e.g., corticosteroids).

Examples of phototherapy for use with secukinumab include treatment with psoralen + UVA (PUVA) or treatment with UVB (with or without tar).

Examples of psoriasis agents used in systemic treatment for use with secukinumab include retionoids such as Acitretin (e.g., Soriatane ^{®}), cyclosporine, methotrexate, hydroxyurea (e.g., Hydrea^{®}), isotretinoin, mycophenolate mofetil, mycophenolic acid, sulfasalazine, 6-thioguanine, fumarates (e.g, dimethylfumarate and fumaric acid esters), azathioprine, corticosteroids, leflunomide, tacrolimus, T-cell blockers (such as Amevive^{®} (alefacept) and Raptiva^{®} (efalizumab), tumor necrosis factor-alpha (TNF-alpha) blockers (such as Enbrel^{®} (etanercept), Humira^{®} (adalimumab), Remicade^{®} (infliximab) and Simponi^{®} (golimumab)) and interleukin 12/23 blockers (such as Stelara^{®} (ustekinumab), tasocitinib, and briakinumab.

Additional psoriasis agents for use in combination with secukinumab during treatment of psoriasis include apremilast, mometasome, voclosporin, ketokonazol, Neuroskin Forte, recombinant human interleukin-10, voclosporin, MK-3222, tofacitinib, VX-765, MED-I545, fluphenazine decanoate, acetomuinophn, bimosiamose cream, doxycycline, vancomycin, AbCm168, Vitamin D3, RO5310074, fludarabine Calcipotriol and hydrocortisone (LEO 80190), LE80185 (Taclonex^{®} Scalp topical suspension/Xamiol^{®} gel), Focetria (Monovalent MF59-Adjuvanted vaccine, tgAAC94 gene therapy vector, Apremilast, Capsaicin, Psirelax, ABT-874 (anti IL-12), IDEC-114, MEDI-522, INCB018424 phosphate cream, LE29102, BMS 587101, CD 2027, CRx-191, 8-methoxypsoralen or 5- methoxypsoralen, Bicillin L-A, LY2525623, INCB018424, LY2439821, CEP-701, CC-10004, certolizumab (CZP), GW786034 (pazopanib), doxycycline Curcuminoids C3 Complex, NYC 0462, RG3421, hOKT3gamma1(Ala-Ala), BT061, teplizumab, Chondroitin sulphate, CNTO 1275, monoclonal antibody to IL-12p40 and IL-23 p40 subunits, BMS-582949, MK0873, MEDI-507, M518101, ABT-874, AMG 827, AN2728, AMG 714, AMG 139, PTH (1-34), U0267 Foam, CNTO 1275, QRX-101, CNTO 1959, LEO 22811, Imiquimod, CTLA4Ig, Alga Dunaliella Bardawil, AS101 Cream, pioglitazone, pimecrolimus, ranibizumab, Zidovudine CDP870 (Certolizumab pegol), Onercept (r-hTBP-1), ACT-128800, 4,4-dimethyl-benziso-2H-selenazine, CRx-191, CRx-197, doxercalciferol, LEO 19123 Cream (calcipotriol plus LEO 80122), LAS 41004, WBI-1001, tacrolimus, RAD001, rapamycin, rosiglitazone, pioglitazone, ABT-874, Aminopterin, AN2728, CD2027, ACT-128800, mometasone furoate, CT 327, clobetasol + LCD, BTT1023, E6201, topical vitamin B12, INCB018424 Phosphate Cream, Xamiol gel, IP10.C8, BFH772, LEO 22811, Fluphenazine, MM-093, Clobex, SCH 527123, CF101, SRT2104, BIRT2584, CC10004, Tetrathiomolybdate, CP-690,550, U0267, ASP015K, VB-201, Acitretin (also called U0279), RWJ-445380, Psoralait, Clobetasol propionate, botulinum toxin type A, alefacept, erlotinib, BCT194, Ultravate Ointment, Roflumilast, CNTO 1275, halobetasol, ILV-094, CTA018 cream, COL-121, MEDI-507, AEB071. Additional agents for use in combination with secukinumab during treatment of psoriasis include IL-6 antagonists, CD20 antagonistis, CTLA4 antagnonists, IL-17 antagonists, IL-8 antagnoists, IL-21 antagonistis, IL-22 antagonist, VGEF antagnosits, CXCL antagonists, MMP antagonists, defensin antagonists, IL-1beta antagonists, and IL-23 antagonists (e.g., receptor decoys, antagonistic antibodies, etc.). A skilled artisan will be able to discern the appropriate dosages of the above agents for co-delivery with secukinumab.

The IL-17 antagonist secukinumab is conveniently administered subcutaneously. Also contemplated is subcutaneous (SC) therapy using a pharmaceutical composition of the present disclosure. The health care provider will decide on the appropriate duration of SC therapy and the timing of administration of the therapy, using the pharmaceutical composition of the present disclosure.

The IL-17 antagonist secukinumab is administered to the patient SC, at 150 mg - 300 mg (e.g., 150 mg, 300 mg) weekly during weeks 0, 1, 2, and 3, and thereafter administered to the patient SC, at 150 mg - 300 mg (e.g., 150 mg, 300 mg) monthly, beginning during week 4. In this manner, the patient is dosed SC with 150 mg - 300 mg (e.g., 150 mg, 300 mg) of the IL-17 antagonist (secukinumab) during weeks 0, 1, 2, 3, 4, 8, 12, 16, 20, etc.

The timing of dosing is generally measured from the day of the first dose of secukinumab (which is also known as "baseline"). However, health care providers often use different naming conventions to identify dosing schedules, as shown in **Table 2.**

**Table 2: Common naming conventions for dosing regimens. Bolded items refer to the naming convention used herein.**

| Week | **0**/1 | **1**/2 | **2**/3 | **3**/4 | **4**/5 | **5**/6 | **6**/7 | **7**/8 | **8**/9 | **9**/10 | **10**/11 | etc |
|---|---|---|---|---|---|---|---|---|---|---|---|---|
| 1^{st} day of week | 0/**1** | 7/**8** | 14/**15** | 21/**22** | 28/**29** | 35/**36** | 42/**43** | 49/**50** | 56/**57** | 63/**64** | 70/**71** | etc. |

Notably, week zero may be referred to as week one by some health care providers, while day zero may be referred to as day one by some health care providers. Thus, it is possible that different physicians will designate, e.g., a dose as being given during week 3 / on day 21, during week 3 / on day 22, during week 4 / on day 21, during week 4 / on day 22, while referring to the same dosing schedule. For consistency, the first week of dosing will be referred to herein as week 0, while the first day of dosing will be referred to as day 1. However, it will be understood by a skilled artisan that this naming convention is simply used for consistency and should not be construed as limiting, i.e., weekly dosing is the provision of a weekly dose of the IL-17 antibody regardless of whether the physician refers to a particular week as "week 1" or "week 2". Moreover, the antibody is administered during week 0, 1, 2, 3, 4 8, 12, 16, 20, etc. Some providers may refer to this regimen as weekly for five weeks and then monthly (or every 4 weeks) thereafter, beginning during week 8, while others may refer to this regimen as weekly for four weeks and then monthly (or every 4 weeks) thereafter, beginning during week 4. Thus, it will be appreciated by a skilled artisan that administering a patient an injection at weeks 0, 1, 2 and 3, followed by once monthly dosing starting at week 4 is the same as: 1) administering the patient an injection at weeks 0, 1, 2, 3, and 4, followed by once monthly dosing starting at week 8; 2) administering the patient an injection at weeks 0, 1, 2, 3 and 4 followed by dosing every 4 weeks; and 3) administering the patient an injection at weeks 0, 1, 2, 3 and 4 followed by monthly administration.

As used herein, the phrase "formulated at a dosage to allow [route of administration] delivery of [a designated dose]" is used to mean that a given pharmaceutical composition can be used to provide a desired dose of the IL-17 antagonist, i.e., the IL-17 antibody secukinumab via a designated route of administration (SC). As an example, if a desired subcutaneous dose is 300 mg, then a clinician may use 2 ml of the IL-17 antibody formulation having a concentration of 150 mg/ml, 1 ml of the IL-17 antibody formulation having a concentration of 300 mg/ml, 0.5 ml of the IL-17 antibody formulation having a concentration of 600 mg/ml, etc. In each such case, these IL-17 antibody formulations are at a concentration high enough to allow subcutaneous delivery of the IL-17 antibody. Subcutaneous delivery typically requires delivery of volumes of less than or equal to about 2 ml, preferably a volume of about 1 ml or less. Preferred formulations are liquid pharmaceutical compositions comprising about 25 mg/mL to about 150 mg/mL secukinumab, about 10 mM to about 30 mM histidine pH 5.8, about 200 mM to about 225 mM trehalose, about 0.02% polysorbate 80, and about 2.5 mM to about 20 mM methionine.

As used herein, the phrase "container having a sufficient amount of the IL-17 antagonist to allow delivery of [a designated dose]" is used to mean that a given container (e.g., vial, pen, syringe) has disposed therein a volume of the IL-17 antagonist (e.g., as part of a pharmaceutical composition) that can be used to provide a desired dose. As an example, if a desired dose is 150 mg, then a clinician may use 2 ml from a container that contains the IL-17 antibody formulation with a concentration of 75 mg/ml, 1 ml from a container that contains the IL-17 antibody formulation with a concentration of 150 mg/ml, 0.5 ml from a container contains the IL-17 antibody formulation with a concentration of 300 mg/ml, etc. In each such case, these containers have a sufficient amount of the IL-17 antagonist to allow delivery of the desired 150 mg dose.

The patient has moderate to severe new-onset plaque-type psoriasis. In some embodiments, the patient has not been previously treated with a systemic treatment for psoriasis. In some embodiments, the patient is biological-naive. In some embodiments, the patient is topical-naive. In some embodiments, the patient has not been previously treated with phototherapy for psoriasis.

The patient is administered 150 mg - 300 mg of the IL-17 antibody by subcutaneous (SC) injection at weeks 0, 1, 2 and 3, followed by once monthly dosing starting at week 4. In some embodiments, the patient is administered 150 mg of the IL-17 antibody by SC injection at weeks 0, 1, 2 and 3, followed by once monthly dosing starting at week 4. In some embodiments, the patient is administered 300 mg of the IL-17 antibody by SC injection at weeks 0, 1, 2 and 3, followed by once monthly dosing starting at week 4.

In some embodiments, the step of administering the IL-17 antibody every four weeks provides an average steady-state trough level of the IL-17 antibody between about 5 µg/ml - about 70 µg/ml, about 5 µg/ml - about 33 µg/ml, or about 11 µg/ml - about 70 µg/ml.

The IL-17 antibody is secukinumab.

In some embodiments, the dose of the IL-17 antibody is 300 mg if the patient weighs > or ≥ 90kg. In some embodiments, the dose of the IL-17 antibody is 300 mg if the patient weighs > or ≥ 100kg. In some embodiments, the dose of the IL-17 antibody is 150 mg if the patient weighs < or ≤ 90kg. In some embodiments, the dose of the IL-17 antibody is 150 mg if the patient weighs < or ≤ 100kg.

In some embodiments, the dose of the IL-17 antibody (secukinumab) is 150 mg, the IL-17 antibody (secukinumab) is comprised in a liquid pharmaceutical formulation, 1 ml of the pharmaceutical formulation is disposed within a pre-filled syringe, injection pen, or auto injector, which is disposed within a kit, wherein the kit further comprises instructions for use. In some embodiments, the dose of the IL-17 antibody (secukinumab) is 150 mg, the IL-17 antibody (secukinumab) is comprised in a liquid pharmaceutical formulation at a concentration of 150 mg/ml, 1 ml of the pharmaceutical formulation is disposed within an autoinjector, which is disposed within a kit, wherein the kit further comprises instructions for use.

In some embodiments, the dose of the IL-17 antibody (secukinumab) is 300 mg, the IL-17 antibody (secukinumab) is comprised in a liquid pharmaceutical formulation at a concentration of 150 mg/ml, the pharmaceutical formulation is disposed within pre-filled syringes, injection pens, or autoinjectors, which are disposed within a kit, wherein the kit further comprises instructions for use. In some embodiments, the dose of the IL-17 antibody is 300 mg, the IL-17 antibody (secukinumab) is comprised in a liquid pharmaceutical formulation at a concentration of 150 mg/ml, 2 ml of the pharmaceutical formulation is disposed within an auto injector, which is disposed within a kit, wherein the kit further comprises instructions for use.

In some embodiments, the patient has a baseline IGA score of ≥ 3. In some embodiments the patient has a baseline PASI score of ≥ 12. In some embodiments, the patient has a baseline BSA of ≥ 10%. Patients' PASI and IGA scores in response to treatment with secukinumab may be found in Langley et al. (2014) N Engl J Med 371:326-38.

In some embodiments, at least 60%, at least 67%, at least 70%, at least 71%, at least 77%, or at least 81% of patients (e.g., adult patients) treated according to the disclosed methods achieve PASI 75 at week 12.

In some embodiments, at least 50%, at least 51%, at least 62%, or at least 65% of patients (e.g., adult patients) patients treated according to the disclosed methods a response of 0 or 1 on the modified investigator's global assessment (IGA) at week 12.

In some embodiments, at least 35%, at least 39%, at least 41%, at least 54%, or at least 59% of patients (e.g., adult patients) treated according to the disclosed methods achieve PASI 90 at week 12.

In some embodiments, at least 10%, at least 12%, at least 14%, at least 24%, or at least 28% of patients (e.g., adult patients) treated according to the disclosed methods achieve PASI 100 at week 12.

Additionally disclosed herein is secukinumab for use in methods of treating a patient having moderate to severe new-onset plaque-type psoriasis, comprising administering the patient 150 mg of secukinumab by subcutaneous injection at weeks 0, 1, 2 and 3, followed by once monthly dosing starting at week 4, wherein the patient is biological-naive. Additionally disclosed herein is secukinumab for use in methods of treating a patient having moderate to severe new-onset plaque-type psoriasis, comprising administering the patient 300 mg of secukinumab by subcutaneous injection at weeks 0, 1, 2 and 3, followed by once monthly dosing starting at week 4, wherein the patient is biological-naive.

### Kits

The disclosure also encompasses kits for treating new-onset plaque-type psoriasis. Such kits comprise the IL-17 antagonist secukinumab (e.g., in liquid or lyophilized form) or a pharmaceutical composition comprising the IL-17 antagonist (described *supra).* Additionally, such kits may comprise means for administering the IL-17 antagonist (e.g., an auto injector, a syringe and vial, a prefilled syringe, a prefilled pen) and instructions for use. These kits may contain additional therapeutic psoriasis agents (described *supra)* for treating new-onset plaque-type psoriasis, e.g., for delivery in combination with the enclosed IL-17 antagonist secukinumab. Such kits may also comprise instructions for administration of the IL-17 antagonist (secukinumab) to treat the new-onset plaque-type psoriasis patient. Such instructions may provide the dose (e.g., 150 mg, 300 mg), route of administration (SC), and dosing regimen (weekly during week 0, 1, 2, and 3 and thereafter monthly (every 4 weeks), beginning during week 4; etc.) for use with secukinumab.

The phrase "means for administering" is used to indicate any available implement for systemically administering a drug to a patient, including, but not limited to, a pre-filled syringe, a vial and syringe, an injection pen, an autoinjector, an IV drip and bag, a pump, etc. With such items, a patient may self-administer the drug (i.e., administer the drug without the assistance of a physican) or a medical practitioner may administer the drug.

### General

In the specification and the appended claims, the singular forms include plural referents unless the context clearly dictates otherwise. Unless defined otherwise, all technical and scientific terms used herein have the same meaning as commonly understood by one of ordinary skill in the art to which this disclosure belongs.

### EXAMPLES

### Example 1: Imiquimod Skin and Ear inflammation

Imiquimod is used for the topical treatment of genital and perineal warts caused by the human papilloma virus. The clinical indications for this therapy have further been expanded to include treatment of other virus-associated skin abnormalities as well as pre-cancerous and cancerous skin lesions such as actinic keratoses and superficial basal cell carcinomas. Clinically, it was found that imiquimod can exacerbate psoriasis in patients whose disease was previously well-controlled during topical treatment of actinic keratoses and superficial basal cell carcinomas. Imiquimod induced exacerbation of psoriasis occurs at both the treated area and, interestingly, also at distant skin sites that were previously unaffected by the disease. Thus, treatment of mice with imiquimod cream, which produces skin lesions similar to psoriasis, can be used to investigate putative anti-psoriasis therapies at an early stage of the disease process. (van der Fits et al. (2009) J. Immunol 182:5836-5845).

### A. Systemic Delivery

Female Balb/c mice (8-10 weeks old) were used for the skin inflammation model. Cream containing 5% imiquimod or vehicle cream was applied daily to the shaved backs of the mice (total imiquimod dose, 12.5mg per mouse). The skin thickness on the back was measured daily using digital callipers.

Anti-IL-17 Ab was administered, by intraperitoneal injection, on days -3, 0, 4, 7 and 11 relative to the first application of the cream. The skin thickness was calculated as % change vs day 0 (baseline) for each time point to give Area Under the Curve (AUC) graphs for vehicle cream, imiquimod cream and Ab treatment groups. The percentage inhibitions of the individual animals in each treatment group AUCs were calculated vs. the imiquimod group AUC (0% inhibition) using an Excel spreadsheet.

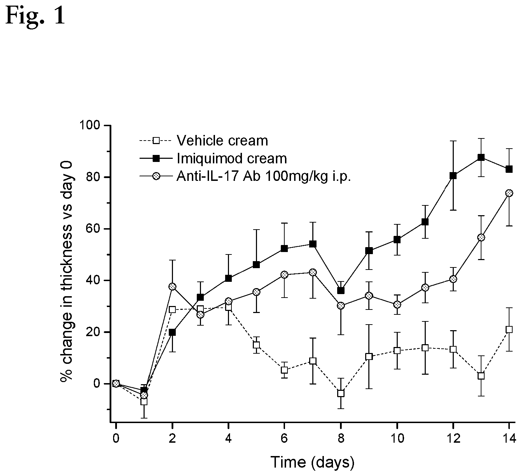

As shown in **Figure 1**, treatment with anti-IL-17 Ab, dosed at 100mg/kg i.p. on days -3, 0, 4, 7 and 11, inhibited skin swelling AUC_{[0-14 days]} by 22.94±12.25%. Thus, early systemic treatment of imiquimod-induced skin thickness with an IL-17 antibody reduces skin thickness in this model.

### B. Local Delivery

Female Balb/c mice (8-10 weeks old) were used for the ear inflammation model. Cream containing 5% imiquimod was applied daily to both ears of the mice (total imiquimod dose, 12.5 mg per mouse). The ear thickness of both ears was measured daily using digital callipers and averaged.

Anti-IL-17 Ab was administered, as a single dose by subcutaneous injection at 100mg/kg, on day -3 relative to the first application of the cream. The ear thickness was calculated as % change vs. day 0 (baseline) for each time point to give Area Under the Curve (AUC) graphs for imiquimod cream and Ab treatment groups. The AUC percentage inhibitions of the individual animals in each treatment group were calculated vs the imiquimod group AUC (0% inhibition) using an Excel spreadsheet.

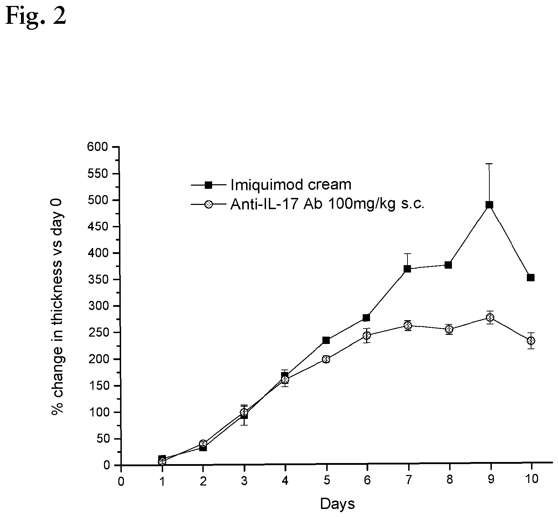

As shown in **Figure 2**, treatment with anti-IL-17 Ab, dosed at 100 mg/kg s.c. on day -3 only, inhibited the ear swelling AUC_{[0-10 days]} by 25.50±2.28%. * p<0.05, unpaired t-test. Thus, early local treatment of imiquimod-induced ear thickness with an IL-17 antibody reduces ear thickness in this model.

Together, the data from the imiquimod model studies suggest that early treatment of new-onset psoriasis with an IL-17 antibody (delivered locally or systemically) may reduce the inflammation (e.g., thickness and/or swelling) associated with the plaque-type skin lesions occurring during psoriasis.

### Example 2: IL-23-Induced Ear Swelling

It has been proposed that IL-23, a cytokine driving the development of IL-17- and IL-22-producing Th17 cells, is functionally involved in the pathogenesis of psoriasis. (See, e.g., van der Fits et al. (2009) J. Immunol 182:5836-5845). Expression of IL-23 is increased in psoriatic skin lesions, and increased numbers of Th17 cells are present. Intradermal injection of IL-23 into mouse skin results in erythema, a mixed inflammatory infiltrate and epidermal hyperplasia, as well as swelling at the injection site after repeated injections. Since both IL-23 and IL-17 have been found to be critical in the development of psoriasis, the IL-23 ear injection model in mice can also be used as a simple and rapid method to investigate therapies which may be useful in the treatment of early psoriasis.

Female Balb/c mice (~20g) were injected i.d. in the right ear pinna with 1µg of IL-23 in 10 µl PBS and with 10µl of PBS alone into the left ear pinna (control ear) on day 0. The ear injections with IL-23 and PBS were repeated on days 2, 5 and 7. Ear thickness was measured using digital callipers, on days 0, 5, 7 and 8.

Anti-IL-17 Ab or isotype control Ab treatment was given at 30mg/kg i.p. as a single dose one day prior to the first ear injections on day 0. Right ear swelling was calculated as a ratio of left ear swelling, and the R/L ear swelling ratio plotted against time to give Area Under the Curve (AUC) graphs for control and treatment groups. The AUC percentage inhibitions of the individual animals in each treatment group were calculated vs. the control group AUC (0% inhibition) using an Excel spreadsheet.

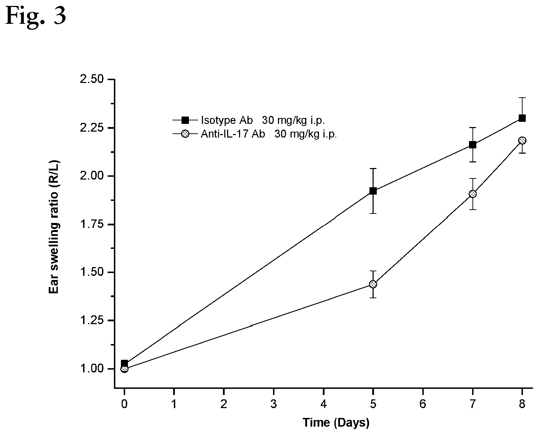

As shown in **Figure 3**, treatment with anti-IL-17 Ab, dosed at 30mg/kg i.p. on day -1 only, inhibited the ear swelling AUC[0-8 days] by 38.57±3.98%. *** p<0.001, paired t-test. This suggests that early treatment of new-onset psoriasis with an IL-17 antibody may reduce the inflammation (e.g., swelling) associated with the plaque-type skin lesions occurring during psoriasis.

### Example 3: Analysis of the Psoriasis Patients' Responses to Treatment with Secukinumab by Disease Duration

Details of the design and results of two phase 3, double-blind, 52-week trials, ERASURE (Efficacy of Response and Safety of Two Fixed Secukinumab Regimens in Psoriasis; CAIN457A2302) and FIXTURE (Full Year Investigative Examination of Secukinumab vs. Etanercept Using Two Dosing Regimens to Determine Efficacy in Psoriasis; CAIN457A2303) are presented in Langley et al. (2014) N Engl J Med 371:326-38. The proportion of patients who met the criterion for PASI 75 at week 12 was higher with each secukinumab dose than with placebo or etanercept: in the ERASURE study, PASI 75 rates were 81.6% with 300 mg of secukinumab, 71.6% with 150 mg of secukinumab, and 4.5% with placebo; in the FIXTURE study, the rates were 77.1% with 300 mg of secukinumab, 67.0% with 150 mg of secukinumab, 44.0% with etanercept, and 4.9% with placebo (P<0.001 for each secukinumab dose vs. comparators). The proportion of patients with a response of 0 or 1 on the modified investigator's global assessment (IGA) at week 12 was higher with each secukinumab dose than with placebo or etanercept: in the ERASURE study, the rates were 65.3% with 300 mg of secukinumab, 51.2% with 150 mg of secukinumab, and 2.4% with placebo; in the FIXTURE study, the rates were 62.5% with 300 mg of secukinumab, 51.1% with 150 mg of secukinumab, 27.2% with etanercept, and 2.8% with placebo (P<0.001 for each secukinumab dose vs. comparators). The rates of infection were higher with secukinumab than with placebo in both studies and were similar to those with etanercept.

The ERASURE and FIXTURE data was reanalyzed to determine whether the effect of secukinumab 150 mg and 300 mg on PASI 75, 90 and 100 and IGA0/1 response rates was different for subgroups of patients at different stages in their psoriasis disease career. The following three subgroups were analyzed: subjects at time of trial enrollment that had been diagnosed with moderate to severe psoriasis for ≤2 years, 2 - ≤10 years and ≥10 years. A summary of time since first diagnosis of psoriasis (years) for the patients in ERASURE and FIXTURE is given below in **Table 3:**

**Table 3 - Disease history and baseline disease characteristics of the induction treatment group (safety set).**

| Background Characteristics | AIN457 150 mg N=692 | AIN457 300 mg N=690 | Placebo N=694 |
|---|---|---|---|
| Time since first diagnosis of psoriasis (years) | | | |
| n | 692 | 690 | 694 |
| Mean | 17.948 | 17.025 | 17.490 |
| SD | 12.4643 | 12.0248 | 12.2314 |
| Minimum | 0.51 | 0.49 | 0.51 |
| Q1 | 8.585 | 7.526 | 7.844 |
| Median | 15.413 | 14.676 | 15.069 |
| Q3 | 25.021 | 23.299 | 25.205 |
| Maximum | 69.01 | 61. 48 | 68.13 |

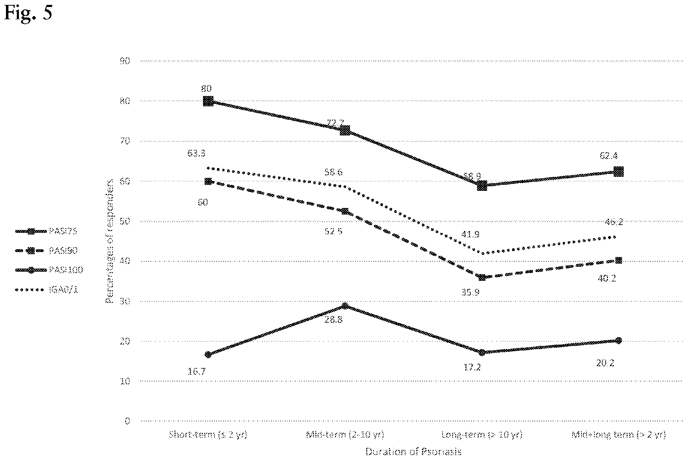

As shown in **Figure 5**, at week 52, for secukinumab 150 mg treatment, except PASI 100, the percentage of response is highest in the short-term subgroup (i.e., patients who had been diagnosed with psoriasis for ≤2 years prior to treatment). This was consistently followed by the mid-term patient subgroup (i.e., patients who had been diagnosed with psoriasis for 2 - ≤10 years) and the long-term patient subgroup (i.e., patients who had been diagnosed with psoriasis for >10 years). Thus, following treatment with 150 mg secukinumab, there is a greater rate of responders amongst short-term patients in achieving PASI 75/90 and IGA 0/1 improvements compared to patients with more advanced disease. Of those patients having psoriasis duration for ≤2 years prior to treatment (n=30), 14 had previously been treated with non-biological systemic therapy; all patients were biological-naive and topical naive. Further analysis showed that of patients having psoriasis duration for ≤ 1 years prior to treatment (n=7), 2 had previously been treated with non-biological systemic therapy; all patients were biological-naive and topical naive.

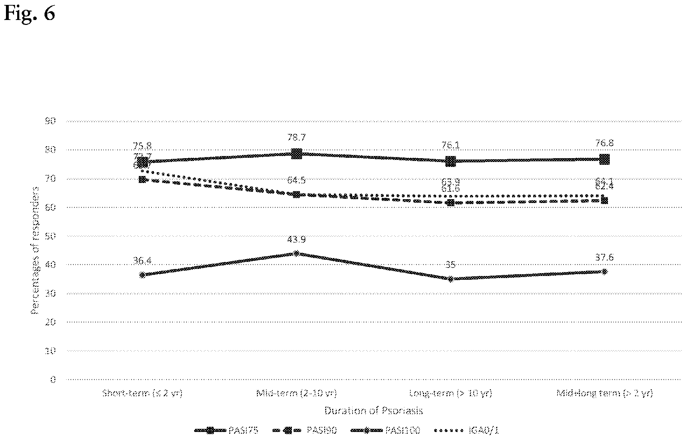

As shown in **Figure 6****,** at week 52, for secukinumab 300 mg treatment, for PASI 75 and PASI 100, the difference between the rate of responders in short-term (≤ 2 yr) versus not-short-term (> 2 yr) is only about 1.1%. However, for PASI 90 and IGA 0/1, the percent of responders in the short-term subgroup is higher than in the mid- and long-term subgroups. Thus, following treatment with 300 mg secukinumab, there is a greater rate of responders amongst short-term patients in achieving PASI 90 and IGA 0/1 improvements compared to patients with more advanced disease. Of those patients having psoriasis duration for ≤2 years prior to treatment (n=33), 22 had previously been treated with non-biological systemic therapy; all patients were biological-naive and topical naive. Further analysis showed that of patients having psoriasis duration for ≤ 1 years prior to treatment (n=11), 8 had previously been treated with non-biological systemic therapy; all patients were biological-naive and topical naive.

A similar analysis was performed for patients in study CAIN457A2317, which used 300 mg secukinumab to treat moderate to severe psoriasis. Interestingly, in this analysis, the rate of improvement achieved by the short-term subgroup was comparable to the rate observed in patients with more advanced disease (data not shown). However, it must be noted that in CAIN457A2317 there were only 15 patients having psoriasis duration for ≤2 years prior to treatment, making comparisons between the subgroups difficult for this trial. Of those patients having psoriasis duration for ≤2 years prior to treatment (n=15), 8 had previously been treated with non-biological systemic therapy, and one had previously been treated with biological systemic therapy. Further analysis showed that of patients having psoriasis duration for ≤ 1 years prior to treatment (n=5), 4 had previously been treated with non-biological systemic therapy; all patients were biological-naive.

### Example 4: CAIN457A2322 - A randomized, multicenter study to evaluate the effect of secukinumab administered to patients suffering from new-onset moderate to severe plaque psoriasis.

| Protocol number | | CAIN457A2322 |
|---|---|---|
| Title | | A randomized, multicenter STudy to evaluate the Effect of secukinumab 300 mg s.c. administered during 52 weeks to patients suffering from new-onset moderate to severe plaque Psoriasis as early Intervention compared to standard treatment with narrow-band UVB (STEPIn study) |
| Brief title | | Study of the efficacy of early intervention with secukinumab 300 mg s.c. compared to narrow-band UVB in patients with new-onset moderate to severe plaque psoriasis |
| Sponsor and clinical phase | | Novartis |
| | | Phase IV |
| Investigation type | | Drug |
| Study type | | Interventional |
| Purpose and rationale | | The purpose of this study is to determine whether early intervention with subcutaneous (s.c.) secukinumab 300 mg in patients with new-onset moderate to severe plaque psoriasis may lead to prolonged symptom-free periods by preventing reactivation of old lesions or ultimately totally hindering the occurrence of new lesions, i.e., changing the natural course of the disease (Main Study). |
| Primary objective | | To demonstrate that early treatment with secukinumab 300 mg s.c. (Arm A1) is superior to standard of care treatment with nb-UVB (Arm B1) in patients with new-onset moderate to severe plaque psoriasis with respect to patients achieving ≥ 90% improvement (reduction) in psoriasis area and severity index (PASI 90) response at Week 52. |
| Secondary objectives | | ***Key secondary objective*** |
| | | To evaluate the superiority of early treatment with secukinumab (Arm A1) versus nb-UVB (Arm B1) based on the proportion of all randomized patients who achieve at least PASI 90 at Week 104. |
| | | ***Additional secondary objective*** |
| | | To evaluate the effects of early treatment with secukinumab (Arm A1) compared with nb-UVB (Arm B1) based on the proportion of all randomized patients who achieve at least investigator's global assessment (IGA mod 2011) of 0 or 1 at Week 52 and at Week 104. |
| Study design Population | The design consists of the Main Study involving all patients in Arms A1 (A1a and A1b) and B1 (B1a and B1b) and a Mechanistic Sub-study, which comprises 5 treatment arms (A1b, A2, B1b, C1, and C2). | |
| | The Main Study will be multicenter, randomized, 2-treatment-arm (secukinumab and nb-UVB), parallel-group and open-label. | |
| | The overall study population (Main Study and Mechanistic Sub-study) will consist of a total of 196 male and female patients aged between 18 and 50 years inclusive. | |
| | ***Main Study*** | |
| | The Main Study will be conducted in patients with new-onset moderate to severe plaque psoriasis not previously treated with any systemic treatment or phototherapy. | |
| | A total of 160 patients will be randomized to Arm A1 or Arm B1 in approximately 75 sites worldwide. Since a maximum screening failure rate of 20% is expected, approximately 245 patients will be screened. | |
| | ***Mechanistic Sub-study*** | |
| | Any patient who consents can participate in the Mechanistic Sub-study. Patients with new-onset plaque psoriasis will be randomized to Arm A1b, Arm A2, or Arm B1b, those with chronic plaque psoriasis will be randomized to Arm C1 and Arm C2 (12 patients each). For Arm A1b or Arm B1b, the first 12 patients will be included on a first come first serve basis. | |
| Key inclusion criteria | Patients eligible for inclusion in this study must fulfill all of the following criteria: | |
| | | 1. Able to understand and communicate with the investigator, willing and capable to comply with all study procedures, and provide written signed and dated informed consent (personally or by a witness) before any assessment is performed |
| | | 2. Aged 18 to 50 years inclusive |
| | | 3. New-onset plaque psoriasis with appearance of the first psoriasis plaques within the last 12 months before randomization and naive to any systemic treatment and phototherapy (Arms A1, A2 and Arm B1) |
| | | 4. Chronic plaque psoriasis with appearance of the first psoriasis symptoms 5 years or longer and intolerance or inadequate response to phototherapy or any systemic treatment including biologicals, except for IL-17A inhibitors (Arm C1 and Arm C2) |
| | | 5. Moderate to severe plaque psoriasis defined at screening and baseline by PASI ≥ 10, and body surface area (BSA) ≥ 10%, and IGA mod 2011 ≥ 3 |
| Key exclusion criteria | Patients fulfilling any of the following criteria will not be eligible for inclusion in this study: | |
| | | 1. Forms of psoriasis other than plaque-type (e.g., pustular, erythrodermic, guttate, light sensitive, and drug induced) |
| | | 2. Ongoing use of prohibited treatments |
| | | 3. Previous treatment with phototherapy or any systemic treatment |
| | | 4. Pregnant or nursing (lactating) women |
| | | 5. Women of childbearing potential, defined as all women physiologically capable of becoming pregnant, unless they are using effective methods of contraception during the Treatment Epoch or longer if required by locally-approved prescribing information (e.g., 20 weeks in the EU for secukinumab) |
| Study treatment | Secukinumab (AIN457) 300 mg | |
| | Narrow-band UVB | |
| Efficacy assessments | • Body surface area and psoriasis area severity index | |
| | • Investigator's global assessment mod 2011 | |
| | • Subject's assessment of pain, itching and scaling | |
| | • Subject's global assessment of psoriatic disease | |
| Safety assessments | | • Physical examination |
| | | • Vital signs |
| | | • Height and body weight |
| | | • Laboratory evaluations (hematology, clinical chemistry, high-sensitivity C-reactive protein) |
| | | • Electrocardiogram |
| | | • Pregnancy |
| | | • Adverse events |
| Other assessments | | • Evaluation of psoriatic arthritis symptoms |
| | | • Dermatology life quality index |
| | | • Work productivity and activity impairment questionnaire: psoriasis |
| | | • Immunological analysis of skin biopsies |
| | | • Human β-defensin 2 |
| Data analysis | The primary efficacy variable is the proportion of patients who achieve PASI 90 at Week 52. The analysis for the primary objective will be based on the full analysis set. | |
| | For the primary analysis, the following hypothesis testing will be performed: | |
| | H₀₁: psec = p_{nbUVB} versus H_{A1}: psec ≠ p_{nbUVB} The primary analysis method for PASI 90 response at Week 52 will use an exact logistic regression model with treatment as an explanatory variable and baseline PASI score as covariate. | |
| | The key secondary variable is the proportion of all randomized patients who achieve PASI 90 at Week 104. In order to reduce selection bias, all patients who do not achieve PASI 90 at Week 52 will also be included in the analysis at Week 104 using the PASI improvement obtained at Week 104 only. | |
| | For the key secondary analysis, the following hypothesis testing will be performed: | |
| | H₀₂: p*_{sec} = p*_{nbUVB} versus H_{A2}: p*_{sec} ≠ p*_{nbUVB} | |
| Keywords | Psoriasis, IL-17A, secukinumab, narrow-band UVB, PASI | |
| Brief title | Study of the efficacy of early intervention with secukinumab 300 mg s.c. compared to narrow-band UVB in patients with new-onset moderate to severe plaque psoriasis | |
| Investigation type | Drug | |
| Study type | Interventional | |
| Purpose and rationale | The purpose of this study is to determine whether early intervention with subcutaneous (s.c.) secukinumab 300 mg in patients with new-onset moderate to severe plaque psoriasis may lead to prolonged symptom-free periods by preventing reactivation of old lesions or ultimately totally hindering the occurrence of new lesions, i.e., changing the natural course of the disease (Main Study). | |
| Primary objective | To demonstrate that early treatment with secukinumab 300 mg s.c. (Arm A1) is superior to standard of care treatment with nb-UVB (Arm B1) in patients with new-onset moderate to severe plaque psoriasis with respect to patients achieving ≥ 90% improvement (reduction) in psoriasis area and severity index (PASI 90) response at Week 52. | |
| Secondary objectives | **Key secondary objective** | |
| | To evaluate the superiority of early treatment with secukinumab (Arm A1) versus nb-UVB (Arm B1) based on the proportion of all randomized patients who achieve at least PASI 90 at Week 104. | |
| | **Additional secondary objective** | |
| | To evaluate the effects of early treatment with secukinumab (Arm A1) compared with nb-UVB (Arm B1) based on the proportion of all randomized patients who achieve at least investigator's global assessment (IGA mod 2011) of 0 or 1 at Week 52 and at Week 104. | |
| Study design | The design consists of the Main Study involving all patients in Arms A1 (A1a and A1b) and B1 (B1a and B1b) and a Mechanistic Sub-study, which comprises 5 treatment arms (A1b, A2, B1b, C1, and C2). | |
| | The Main Study will be multicenter, randomized, 2-treatment-arm (secukinumab and nb-UVB), parallel-group and open-label. | |
| Population | The overall study population (Main Study and Mechanistic Sub-study) will consist of a total of 196 male and female patients aged between 18 and 50 years inclusive. | |
| | **Main Study** | |
| | The Main Study will be conducted in patients with new-onset moderate to severe plaque psoriasis not previously treated with any systemic treatment or phototherapy. | |
| | A total of 160 patients will be randomized to Arm A1 or Arm B1 in approximately 75 sites worldwide. Since a maximum screening failure rate of 20% is expected, approximately 245 patients will be screened. | |
| | **Mechanistic Sub-study** | |
| | Any patient who consents can participate in the Mechanistic Sub-study. Patients with new-onset plaque psoriasis will be randomized to Arm A1b, Arm A2, or Arm B1b, those with chronic plaque psoriasis will be randomized to Arm C1 and Arm C2 (12 patients each). For Arm A1b or Arm B1b, the first 12 patients will be included on a first come first serve basis. | |
| Key inclusion criteria | Patients eligible for inclusion in this study must fulfill all of the following criteria: | |
| | | 6. Able to understand and communicate with the investigator, willing and capable to comply with all study procedures, and provide written signed and dated informed consent (personally or by a witness) before any assessment is performed |
| | | 7. Aged 18 to 50 years inclusive |
| | | 8. New-onset plaque psoriasis with appearance of the first psoriasis plaques within the last 12 months before randomization and naive to any systemic treatment and phototherapy (Arms A1, A2 and Arm B1) |
| | | 9. Chronic plaque psoriasis with appearance of the first psoriasis symptoms 5 years or longer and intolerance or inadequate response to phototherapy or any systemic treatment including biologicals, except for IL-17A inhibitors (Arm C1 and Arm C2) |
| | | 10. Moderate to severe plaque psoriasis defined at screening and baseline by PASI ≥ 10, and body surface area (BSA) ≥ 10%, and IGA mod 2011 ≥ 3 |
| Key exclusion criteria | Patients fulfilling any of the following criteria will not be eligible for inclusion in this study: | |
| | | 6. Forms of psoriasis other than plaque-type (e.g., pustular, erythrodermic, guttate, light sensitive, and drug induced) |
| | | 7. Ongoing use of prohibited treatments |
| | | 8. Previous treatment with phototherapy or any systemic treatment |
| | | 9. Pregnant or nursing (lactating) women |
| | | 10. Women of childbearing potential, defined as all women physiologically capable of becoming pregnant, unless they are using effective methods of contraception during the Treatment Epoch or longer if required by locally-approved prescribing information (e.g., 20 weeks in the EU for secukinumab) |
| Study treatment | Secukinumab (AIN457) 300 mg | |
| | Narrow-band UVB | |
| Efficacy assessments | • Body surface area and psoriasis area severity index | |
| | • Investigator's global assessment mod 2011 | |
| | • Subject's assessment of pain, itching and scaling | |
| | • Subject's global assessment of psoriatic disease | |
| Safety assessments | | • Physical examination |
| | | • Vital signs |
| | | • Height and body weight |
| | | • Laboratory evaluations (hematology, clinical chemistry, high-sensitivity C-reactive protein) |
| | | • Electrocardiogram |
| | | • Pregnancy |
| | | • Adverse events |
| Other assessments | | • Evaluation of psoriatic arthritis symptoms |
| | | • Dermatology life quality index |
| | | • Work productivity and activity impairment questionnaire: psoriasis |
| | | • Immunological analysis of skin biopsies |
| | | • Human β-defensin 2 |
| Data analysis | The primary efficacy variable is the proportion of patients who achieve PASI 90 at Week 52. The analysis for the primary objective will be based on the full analysis set. | |
| | For the primary analysis, the following hypothesis testing will be performed: H01: psec = pnbUVB versus HA1: psec = pnbUVB | |
| | The primary analysis method for PASI 90 response at Week 52 will use an exact logistic regression model with treatment as an explanatory variable and baseline PASI score as covariate. | |
| | The key secondary variable is the proportion of all randomized patients who achieve PASI 90 at Week 104. In order to reduce selection bias, all patients who do not achieve PASI 90 at Week 52 will also be included in the analysis at Week 104 using the PASI improvement obtained at Week 104 only. | |
| | For the key secondary analysis, the following hypothesis testing will be performed: | |
| | H02: p*sec = p*nbUVB versus HA2: p*sec ≠ p*nbUVB | |
| Keywords | Psoriasis, IL-17A, secukinumab, narrow-band UVB, PASI | |

## Claims

1. Secukinumab for use in treating moderate to severe new-onset plaque-type psoriasis, **characterized in that** 150 mg - 300 mg secukinumab is to be subcutaneously administered to a patient in need thereof at weeks 0, 1, 2, 3, and 4, followed by monthly dosing; wherein the first psoriasis plaque in the patient appeared within 12 months prior to the start of the treatment.

2. Secukinumab for use according to claim 1, wherein the patient has not been previously treated with a systemic treatment for psoriasis.

3. Secukinumab for use according to claim 1 or claim 2, wherein the patient is biological-naive.

4. Secukinumab for use according to any one of claims 1-3, wherein the patient has not been previously treated with phototherapy for psoriasis.

5. Secukinumab for use according to any one of claims 1-4, wherein 300 mg secukinumab is to be subcutaneously administered to the patient at weeks 0, 1, 2, 3, and 4, followed by monthly dosing.

6. Secukinumab for use according to any one of claims 1-4, wherein 150 mg secukinumab is to be subcutaneously administered to the patient at weeks 0, 1, 2, 3, and 4, followed by monthly dosing.

7. Secukinumab for use according to any one of claims 1-6, which is provided in a liquid pharmaceutical composition comprising 25 mg/mL - 150 mg/mL secukinumab, 10 mM - 30 mM histidine pH 5.8, 200 mM - 225 mM trehalose, 0.02% polysorbate 80, and 2.5 mM - 20 mM methionine.

8. Secukinumab for use according to any one of claims 1-4 or 7, wherein 150 mg of secukinumab is to be subcutaneously administered to the patient if the patient weighs < 90 kg, or wherein 300 mg of secukinumab is to be subcutaneously administered to the patient if the patient weighs ≥ 90 kg.

9. Secukinumab for use according to any one of claims 1-4 or 7, wherein 150 mg of secukinumab is to be subcutaneously administered to the patient if the patient weighs < 100 kg, or wherein 300 mg of secukinumab is to be subcutaneously administered to the patient if the patient weighs ≥ 100 kg.

10. Secukinumab for use according to any one of claims 1-9, wherein the patient has a baseline IGA score of ≥ 3, a baseline PASI score of ≥ 12, and/or a baseline BSA of ≥ 10%.

11. Secukinumab for use according to any one of claims 1-10, wherein a population of patients is treated, and wherein at least 60%, at least 67%, at least 70%, at least 71%, at least 77%, or at least 81% of patients achieve at least PASI 75 by week 12 of treatment.

12. Secukinumab for use according to any one of claims 1-11, wherein a population of patients is treated, and wherein at least 50%, at least 51%, at least 62%, or at least 65% of patients achieve a response of 0 or 1 on the modified investigator's global assessment (IGA) by week 12 of treatment.

13. Secukinumab for use according to any one of claims 1-12, wherein a population of patients is treated, and wherein at least 35%, at least 39%, at least 41%, at least 54%, or at least 59% of patients achieve at least PASI 90 response by week 12 of treatment.

## Patentansprüche

1. Secukinumab zur Verwendung bei der Behandlung von mittelschwerer bis schwerer neu aufgetretener Plaque-Psoriasis, **dadurch gekennzeichnet, dass** 150 mg - 300 mg Secukinumab einem Patienten, der dies benötigt, in Wochen 0, 1, 2, 3 und 4 subkutan verabreicht werden sollen, gefolgt von monatlicher Gabe; wobei die erste Psoriasis-Plaque bei dem Patienten innerhalb von 12 Monaten nach Beginn der Behandlung auftrat.

2. Secukinumab zur Verwendung nach Anspruch 1, wobei der Patient zuvor noch nicht mit einer systemischen Behandlung für Psoriasis behandelt wurde.

3. Secukinumab zur Verwendung nach Anspruch 1 oder Anspruch 2, wobei der Patient Biologika-naiv ist.

4. Secukinumab zur Verwendung nach einem der Ansprüche 1-3, wobei der Patient zuvor noch nicht mit Phototherapie für Psoriasis behandelt wurde.

5. Secukinumab zur Verwendung nach einem der Ansprüche 1-4, wobei 300 mg Secukinumab dem Patienten in Wochen 0, 1, 2, 3 und 4 subkutan verabreicht werden sollen, gefolgt von monatlicher Gabe.

6. Secukinumab zur Verwendung nach einem der Ansprüche 1-4, wobei 150 mg Secukinumab dem Patienten in Wochen 0, 1, 2, 3 und 4 subkutan verabreicht werden sollen, gefolgt von monatlicher Gabe.

7. Secukinumab zur Verwendung nach einem der Ansprüche 1-6, das in einer flüssigen pharmazeutischen Zusammensetzung bereitgestellt wird, die 25 mg/ml - 150 mg/ml Secukinumab, 10 mM - 30 mM Histidin pH 5,8, 200 mM - 225 mM Trehalose, 0,02% Polysorbat 80 und 2,5 mM - 20 mM Methionin umfasst.

8. Secukinumab zur Verwendung nach einem der Ansprüche 1-4 oder 7, wobei 150 mg Secukinumab dem Patienten subkutan verabreicht werden sollen, wenn der Patient <90 kg wiegt, oder wobei 300 mg Secukinumab dem Patienten subkutan verabreicht werden sollen, wenn der Patient ≥90 kg wiegt.

9. Secukinumab zur Verwendung nach einem der Ansprüche 1-4 oder 7, wobei 150 mg Secukinumab dem Patienten subkutan verabreicht werden sollen, wenn der Patient <100 kg wiegt, oder wobei 300 mg Secukinumab dem Patienten subkutan verabreicht werden sollen, wenn der Patient ≥100 kg wiegt.

10. Secukinumab zur Verwendung nach einem der Ansprüche 1-9, wobei der Patient einen Baseline-IGA-Score von ≥3, einen Baseline-PASI-Score von ≥12 und/oder einen Baseline-BSA von ≥10% aufweist.

11. Secukinumab zur Verwendung nach einem der Ansprüche 1-10, wobei eine Patientenpopulation behandelt wird, und wobei mindestens 60%, mindestens 67%, mindestens 70%, mindestens 71%, mindestens 77% oder mindestens 81% der Patienten mindestens PASI 75 bis Behandlungswoche 12 erreichen.

12. Secukinumab zur Verwendung nach einem der Ansprüche 1-11, wobei eine Patientenpopulation behandelt wird, und wobei mindestens 50%, mindestens 51%, mindestens 62% oder mindestens 65% der Patienten ein Ansprechen von 0 oder 1 beim modifizierten Globalurteil des Prüfarztes (IGA) bis Behandlungswoche 12 erreichen.

13. Secukinumab zur Verwendung nach einem der Ansprüche 1-12, wobei eine Patientenpopulation behandelt wird, und wobei mindestens 35%, mindestens 39%, mindestens 41%, mindestens 54% oder mindestens 59% der Patienten mindestens PASI-90-Ansprechen bis Behandlungswoche 12 erreichen.

## Revendications

1. Sécukinumab pour une utilisation dans le traitement du psoriasis de type en plaques nouvellement apparu modéré à grave, **caractérisé en ce que** 150 mg à 300 mg de sécukinumab doivent être administrés de manière sous-cutanée à un patient qui en a besoin aux semaines 0, 1, 2, 3 et 4, suivi par un dosage mensuel ; la première plaque de psoriasis chez le patient étant apparue dans les 12 mois avant le début du traitement.

2. Sécukinumab pour une utilisation selon la revendication 1, le patient n'ayant pas été précédemment traité avec un traitement systémique pour le psoriasis.

3. Sécukinumab pour une utilisation selon la revendication 1 ou la revendication 2, le patient étant biologiquement naïf.

4. Sécukinumab pour une utilisation selon l'une quelconque des revendications 1 à 3, le patient n'ayant pas été traité précédemment par photothérapie pour le psoriasis.

5. Sécukinumab pour une utilisation selon l'une quelconque des revendications 1 à 4, 300 mg de sécukinumab devant être administrés de manière sous-cutanée à un patient qui en a besoin aux semaines 0, 1, 2, 3 et 4, suivi par un dosage mensuel.

6. Sécukinumab pour une utilisation selon l'une quelconque des revendications 1 à 4, 150 mg de sécukinumab devant être administrés de manière sous-cutanée à un patient qui en a besoin aux semaines 0, 1, 2, 3 et 4, suivi par un dosage mensuel.

7. Sécukinumab pour une utilisation selon l'une quelconque des revendications 1 à 6, qui est fourni dans une composition pharmaceutique liquide comprenant 25 mg/ml à 150 mg/ml de sécukinumab, 10 mM à 30 mM d'histidine pH 5,8, 200 mM à 225 mM de tréhalose, 0,02 % de polysorbate 80 et 2,5 mM à 20 mM de méthionine.

8. Sécukinumab pour une utilisation selon l'une quelconque des revendications 1 à 4 ou 7, 150 mg de sécukinumab devant être administrés de manière sous-cutanée au patient si le patient pèse < 90 kg, ou 300 mg de sécukinumab devant être administrés de manière sous-cutanée au patient si le patient pèse ≥ 90 kg.

9. Sécukinumab pour une utilisation selon l'une quelconque des revendications 1 à 4 ou 7, 150 mg de sécukinumab devant être administrés de manière sous-cutanée au patient si le patient pèse < 100 kg, ou 300 mg de sécukinumab devant être administrés de manière sous-cutanée au patient si le patient pèse ≥ 100 kg.

10. Sécukinumab pour une utilisation selon l'une quelconque des revendications 1 à 9, le patient ayant un score IGA de ligne de base de ≥ 3, un score PASI de ligne de base de ≥ 12 et/ou un BSA de ligne de base de ≥ 10 %.

11. Sécukinumab pour une utilisation selon l'une quelconque des revendications 1 à 10, une population de patients étant traitée, et au moins 60 %, au moins 67 %, au moins 70 %, au moins 71 %, au moins 77 % ou au moins 81 % de patients atteignant au moins PASI 75 à la semaine 12 de traitement.

12. Sécukinumab pour une utilisation selon l'une quelconque des revendications 1 à 11, une population de patients étant traitée, et au moins 50 %, au moins 51 %, au moins 62 % ou au moins 65 % de patients atteignant une réponse de 0 ou 1 sur l'évaluation globale d'investigateur (IGA) modifiée à la semaine 12 de traitement.

13. Sécukinumab pour une utilisation selon l'une quelconque des revendications 1 à 12, une population de patients étant traitée, et au moins 35 %, au moins 39 %, au moins 41 %, au moins 54 %, ou au moins 59 % de patients atteignant au moins PASI 90 à la semaine 12 de traitement.
